# EUROPEAN PATENT APPLICATION

(11) **EP 0 537 768 A1**
(43) Date of publication of application: **21.04.1993**
(21) Application number: 92117730.9
(22) Date of filing: 16.10.1992
(51) Int. Cl.: A61B 17/34, A61M 25/02

(54) **External laparoscopic sealing means**

(30) Priority: 18.10.1991 US 780713
(71) Applicant: United States Surgical Corporation, Norwalk, Connecticut 06856 (US)
(72) Inventor: Tovey, H. Jonathan, Milford, CT 06460 (US)
(74) Representative: Marsh, Roy David

(57) **Abstract**

A sealing device 10 for use with a cannula or trocar assembly. The device includes a substantially cylindrical member 12 positioned about a cannula or trocar assembly. The cylindrical member has a flange 16 at a distal end such that the flange is positionable against a patient's body. The flange provides a sealing effect such that the gases from the body cavity will not escape, for example, during surgical procedures requiring insufflation.

## Description

### BACKGROUND OF THE INVENTION

### 1. Field of the Invention

The present invention relates to surgical instruments for performing laparoscopic and endoscopic surgical procedures, and more particularly to devices for preventing leakage of gases around the exterior of a cannula particularly after extensive instrument manipulation.

### 2. Discussion of the Prior Art

In recent years, laparoscopic and endoscopic surgical procedures have become increasingly popular for performing major surgical operations. In such a procedure, a small incision or puncture is made in the patient's body to provide access for a tube or a cannula device. The tube or cannula device is inserted into the patient's body to allow for viewing the surgical site and for the insertion of instruments used in performing the surgical procedure. Typically, a trocar device including, for example, an obturator and a cannula is employed. A pointed obturator may assist in penetrating the trocar beyond the body wall of the surgical site. Similarly, an incision may be made using a scalpel or similar device before inserting a blunt obturator. When either obturator is removed, the cannula remains in place to maintain access to the surgical site. Several incisions may be made to provide numerous access ports to the surgical objective. Once the cannulas are in place, various surgical instruments such as scissors, dissectors, retractors or the like, may be inserted by a surgeon to perform the surgery. Typically, a scope device is used to view the area directly, or a miniature camera is used to display the surgical site on a video monitor in the operating room.

The primary benefit of such minimally invasive surgical techniques is the reduction of scarring, and consequently, minimizing damage to surrounding tissue and organs. As a result, recovery time is greatly reduced for the patient.

During a laparoscopic surgical procedure, gas is introduced into the body cavity by means of a pneumoperitoneum needle. The gas inflates the cavity to provide greater access to the surgical area and minimize obstruction during surgery. The trocar assembly is then inserted into the body cavity, usually the abdomen, to a point adjacent the tissue or organ which is the surgical objective. After the obturator is removed from the trocar assembly, the cannula remains in place in the patient's body. Due to the gas insufflation, it is necessary to maintain a desired gas seal at each of the cannulas in position in the body. Ordinarily, a flapper valve in a housing at the proximal end of the cannula prevents the insufflation gas from escaping through the cannula after the obturator is removed. However, gas leakage may also occur from around the incision where the cannula has been positioned. Further, axial, lateral and/or angular movement of the cannulas may result in gas leakage from around the incision, and thereby, negatively affect the surgical procedure.

In order to maintain adequate integrity of the gas seal between a cannula and a body cavity it has been known to provide various mechanisms and devices. Typical devices include a medical device for control of enemata disclosed in U.S. Patent No. 3,459,175 to Miller, which provides a hollow pipe adapted for insertion by its forward end through the anal canal into the lower region of a patient's bowel. The device includes an inflatable annular element of resilient flexible material surrounding the pipe and a U-shaped abutment element including legs and a base. The annular element and the abutment element work in concert to prevent flow of fluid from the bowel.

U.S. Patent No. 4,077,412 to Moossun, as well as U.S. Patent No. 4,627,838 to Cross et al., disclose devices to prevent the inadvertent removal or backing off of the cannula during the surgical procedure and provide some sealing properties, and include an inflatable diaphragm member which is inflated once the cannula is positioned in the body cavity to prevent inadvertent removal of the cannula from the incision until the diaphragm is deflated.

U.S. Patent No. 5,002,557 to Hasson, discloses a device which provides a laparoscopic cannula including a truncated cone-shaped collar surrounding the cannula. An expandable member at the device's distal end is used in concert with the collar to capture tissue therebetween. U.S. Patent No. 3,817,251 to Hasson discloses a conical sleeve which may be adjusted to various positions on the cannula to limit insertion of the cannula to specific depths.

The novel sealing device for use with a cannula or trocar assembly of the present invention obviates the disadvantages encountered in the prior art and provides a simple device for attaching to the cannula or trocar assembly which provides desired sealing between the cannula and the body cavity during a surgical procedure. Alternatively, the device may be constructed integrally with a cannula, or as part of a tissue gripping device attached to a cannula. Moreover, the sealing device provides a non-invasive sealing means that will minimize trauma to the tissue surrounding a cannula. The device of the present invention thus provides the desired gas sealing necessary to perform endoscopic or laparoscopic surgical procedures.

### SUMMARY OF THE INVENTION

The present invention provides a novel sealing device for use with a cannula or similar device. The device maintains a gas seal between the body cavity and cannula after the body cavity has been insufflated during endoscopic or laparoscopic surgical procedures. The sealing device of the present invention may be used with various cannulas, or may be constructed integral with a cannula that is part of a trocar assembly.

The sealing device of the present invention comprises a substantially cylindrical member positioned about a cannula and includes a flange at a distal end which is positionable against a patient's body. Also, preferably, the sealing device is longitudinally adjustable.

In use, the device of the present invention is attached to a cannula. The cannula is inserted into a patient, so that the sealing member of the present invention is resiliently engaged against the patient's body. The sealing device helps avoid desufflation of the body cavity by retarding escaping gases at the incision.

It is further contemplated that the device of the present invention be constructed as part of a trocar assembly which includes a cannula, a tissue gripping member, and an obturator.

### BRIEF DESCRIPTION OF THE DRAWINGS

Preferring embodiments of the invention are described hereinbelow with reference to the drawings wherein:
Fig. 1 illustrates a perspective view of the sealing device according to an embodiment of the invention;
Fig. 2 illustrates a perspective view of the sealing device used with a cannula;
Fig. 3 illustrates a cross-sectional side view of the sealing device used with the cannula shown in Fig. 2;
Fig. 4 illustrates a perspective view of the sealing device used with a trocar assembly;
Fig. 5 illustrates an exploded view of the sealing device used with the trocar assembly shown in Fig. 4;
Fig. 6 illustrates a perspective view of the sealing device used with the trocar assembly shown in Figs. 4 and 5 having a tissue gripping member in an engaged position;
Fig. 7 illustrates an exploded view of the sealing device used with another trocar assembly;
Fig. 8 illustrates a perspective view of the sealing device used with still another trocar assembly; and
Fig. 9 illustrates a perspective view of the sealing device according to another embodiment of the invention.

### DETAILED DESCRIPTION OF THE PREFERRED EMBODIMENTS

A sealing device 10 is provided for use with a tubular member such as a cannula or similar device to deter the escape of gases from the body cavity when, for example, a surgeon is engaging in endoscopic or laparoscopic procedures requiring insufflation of the body cavity.

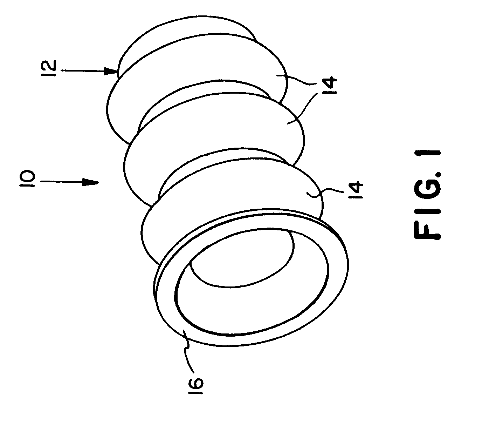

A first embodiment of the sealing device 10 is shown in Fig. 1. The illustrated sealing device comprises a cylindrical member 12. The cylindrical member 12 is shaped similar to bellows in which a plurality of bulbous portions 14 are integrally molded to each other. The cylindrical member 12 includes a flange 16 at its distal end that is positionable against a patient's body.

It is also contemplated that the sealing device's bulbous portions 14 be sealingly attached to each other to allow varying of the number of bulbous portions 14 employed. The sealing device 10 will thereby be longitudinally extended or retracted by varying the number of bulbous portions 14 employed.

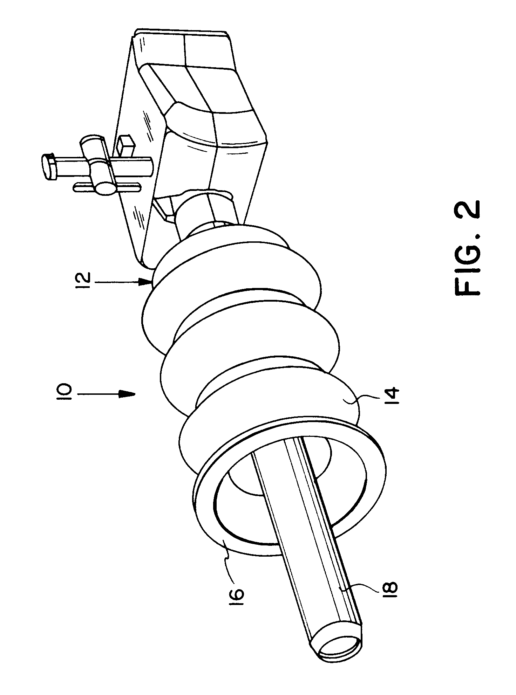

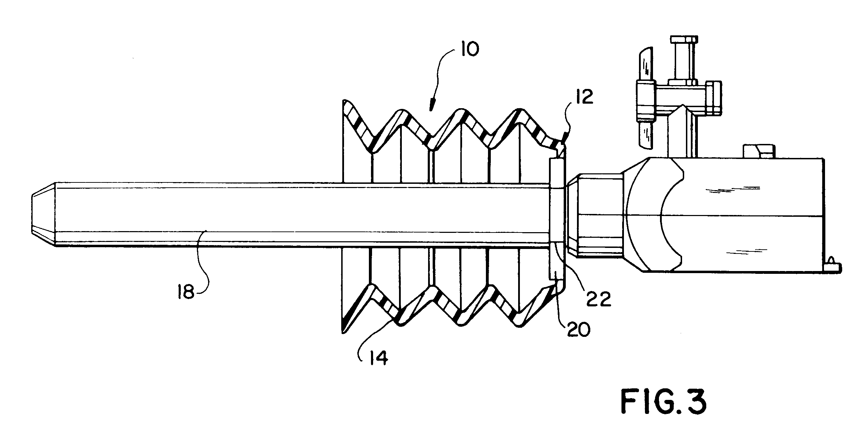

Referring to Figs. 2 and 3, the sealing device 10 in use with or as part of a cannula is shown. As described above, the sealing device 10 includes a cylindrical member 12 having a flange 16 at its distal end that is positionable against a patient's body. However, in Fig. 2 the sealing device 10 is shown positioned about the cannula 18.

As shown in Fig. 3, the inner diameter of the sealing device 10 at its distal end is greater than the diameter of the cannula to provide room for the cannula to change its angular juxtaposition with respect to the patient's body. However, the illustrated sealing device 10 comprises a ridge 20 around the inner circumference of the sealing device's proximal end which preferably cooperates with a receiving indentation or groove 22 at the proximal end of the cannula. It is also contemplated that the inner diameter of the sealing device at its proximal end may be defined by a neck dimensioned to fit securely about the cannula. The sealing device 10, as shown in Figs. 1, 2 and 3, is longitudinally adjustable and resiliently responds when compressed.

In use, the cannula 18 may be part of a trocar assembly 24, as described below. The trocar assembly 24 enables the sealing device 10 of the present invention to be used in concert with a tissue gripping device 26 which may include, for example, malecot wings, an inflatable balloon, or an expandable gripping device.

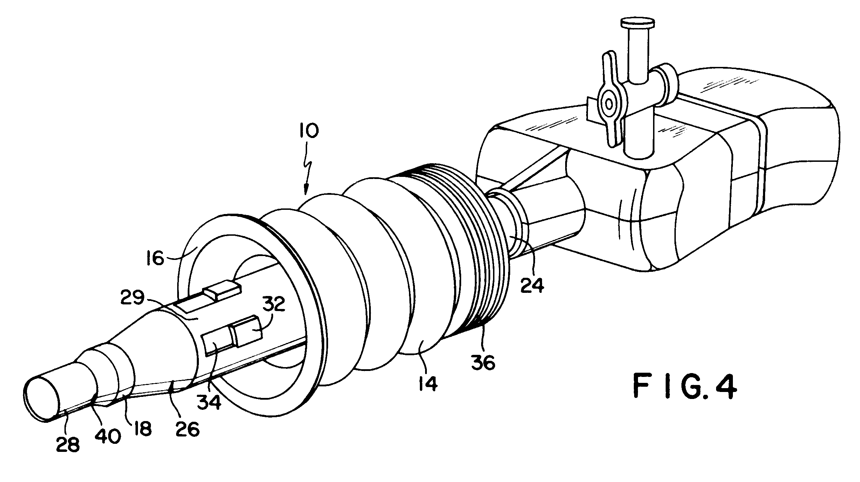

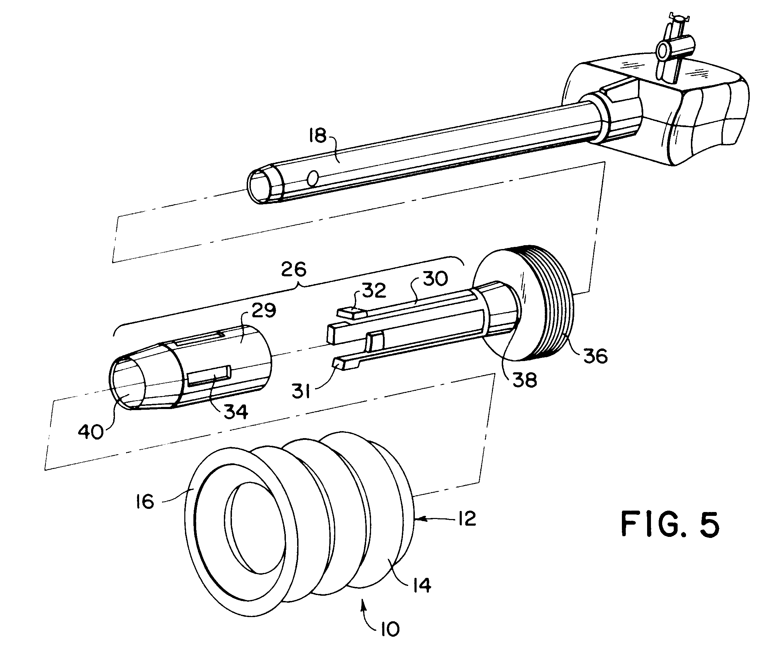

The sealing device 10 in use with or as part of a trocar assembly 24 is shown in Figs. 4, 5 and 6. The sealing device 10 includes a flange 16 at its distal end as in the previous embodiment shown in Figs. 1, 2 and 3. However, in the embodiment shown in Figs. 4, 5 and 6, the sealing device 10 is part of a trocar assembly 24 which preferably includes a cannula 18, a tissue gripping device 26, and an obturator 28. Further, the tissue gripping device 26 includes a housing member 29 fixedly secured to the cannula 18.

The housing member 29 is concentrically secured at a distal end of the cannula 18 by conventional means, such as adhesives, heat staking, sonic welding, set screws, or the like. The tissue gripping device 26 is provided with a plurality of expansion members 30 which surround the cannula and terminate in tissue contact faces 32. The housing member 29 preferably has a truncated conical end, and has a diameter at a proximal end which is greater than an outside diameter of the cannula 18 for accepting the expansion members 30 of the tissue gripping device 26. The expansion members 30 act in concert with slots 34 in the housing member 29 to flex the expansion members 30 outwardly and thereby engage tissue to hold the cannula 18 in place.

Further, the housing member 29 has a generally cylindrical shape which extends from a proximal gripping flange 36, which remains outside the body during the surgical procedure. Adjacent to the gripping flange 36 is a circumferential groove or indentation 38 designed to mate with a ridge 20 at the proximal end of the sealing device. The groove 38 and ridge 20 mate to form a desired seal that allows the sealing device to provide the desired gas seal between the cannula 18 and the body cavity when the flange 16 at the distal end of the sealing device 10 is against a patient's body.

The sealing device 10 may be, for example, constructed of an elastomeric material, which is preferably an elastomer commercially available under the trademark "SANTOPRENE", manufactured by Monsanto.

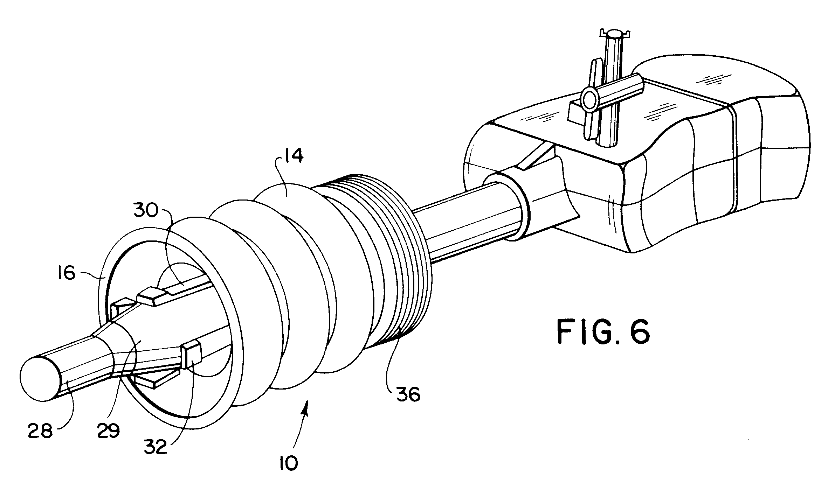

In operation, the sealing device as shown in Figs. 4-6 is used with a trocar assembly 24, which includes an obturator 28 and a cannula body with 18 the tissue gripping device 26 positioned thereon so that the cannula 18 passes through the tissue gripping device 26 and extends through the distal opening 40 of a housing member 29. Referring to Fig. 6, after the cannula 18 is inserted into the body, the tissue gripping member 26 is urged towards the body into the housing member 29. Camming surfaces 31 on each of the finger-like expansion members 30 engage the distal end wall of the slots 34 which flexes the expansion members 30 outwardly into engagement with the tissue at the incision. The cannula 18 is thereby secured in the incision by the outwardly deflected expansion members 30. The sealing device 10 is urged towards the patient's body in concert with the extension of the expansion members 30. The flange 16 contacts the patient's skin providing a desired gas seal for maintaining insufflation pressure within the body cavity in the event there is leakage about the cannula 18 at the incision. The sealing device 10 is designed to remain in substantial contact with a patient's body while accommodating the cannula 18 in varying angular positions with respect to a patient's body. More specifically, the sealing device 10 includes an inside diameter which allows for angular juxtapositioning of the cannula with respect to a patient's body while maintaining the relationship between the flange 16 and a patient's body.

To remove the cannula 18, the tissue gripping member expansion members 30 is withdrawn from the housing member 29 so that the flexible nature of the expansion members 30 draws the expansion member 30 back through the slots. The sealing device 10 is withdrawn in concert with the expansion members 30, and as a result, the flange 16 at the distal end of the sealing device 10 is disengaged from the patient's skin. Then, the entire tissue gripping device 26 and cannula 18 may be withdrawn from the incision.

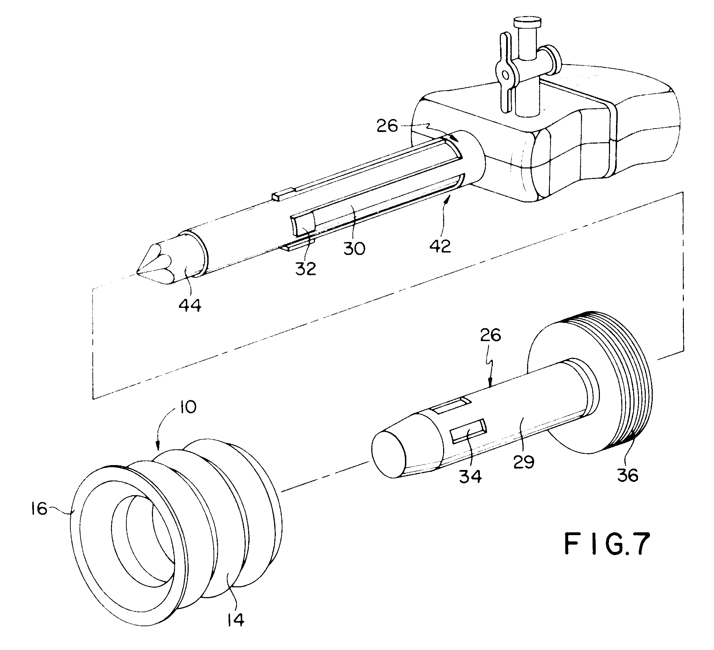

The sealing device 10 in use with or as part of another trocar assembly 42 is shown in Fig. 7 which includes a flange 16 at its distal end as in the previous embodiment shown in Figs. 4-6. However, in the embodiment shown in Fig. 7, the sealing device 10 is part of a trocar assembly 42 where the tissue gripping member 30 is an integral part of a cannula 18, or is fixedly secured thereto by, for example, a set screw, adhesives, etc. Moreover, a pointed obturator 44 is used to create an incision into the body cavity. As discussed above, the tissue gripping device 26 is provided with a plurality of expansion members 30 which surround the cannula 18 and terminate in tissue contact faces 32. A separate housing member 29 is provided which is similar to that described in reference to Figs. 4-6 above. After the trocar assembly 42 is placed in the patient's body, the obturator 44 is removed and the cannula 18 and the sealing device 26 is secured in place as described in reference to Figs. 4-6 above.

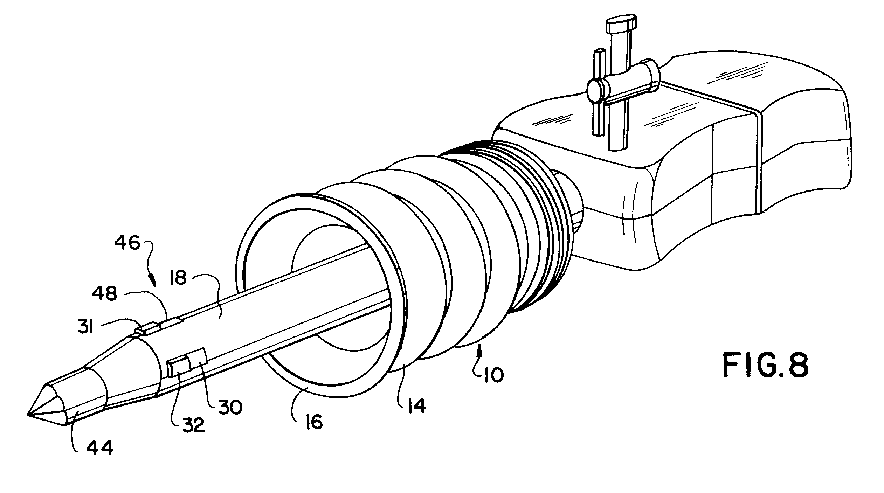

The sealing device 10 in use with or as part of still another trocar assembly 46 is shown in Fig. 8. The sealing device 10 includes a flange 16 at its distal end as in the previous embodiments shown in Figs. 4-7. However, in the embodiment shown in Fig. 8, the sealing device 10 is part of a trocar assembly 46 where the cannula 18 itself has a diameter sufficient to accept the obturator 44 and the tissue gripping device 26 which has elongated flexible finger-like expansion members 30. The cannula 18 itself, preferably, includes a plurality of slots 48 through which the expansion members 30 would extend. The slots 48 preferably are positioned about the circumference of the cylindrical cannula 18. The cannula 18 is inserted into the body using a pointed obturator 44 to penetrate the patient's skin. Preferably, the cannula 18 tapers at its distal end to facilitate penetration into the body such that the inner diameter at the distal end would be slightly greater than the outer diameter of the obturator 44 which passes therethrough. The tissue gripping device 26 is substantially identical to that described in the previous embodiments, where a plurality of expansion members 30 extend into the cannula 18 terminating in camming surfaces 31 that engage the distal end walls of the slots to flex the tissue contacting surfaces 30 outwardly securing the cannula 18 in the tissue at the incision. The obturator 44 is then removed so that the cannula 18 can be used for endoscopic or laparoscopic activities. To remove the tissue gripping device 26, the expansion members 30 are slid rearwardly away from the body to withdraw the expansion members 30 back into the cannula 18 so that the cannula 18 may be removed. As described above, the sealing device 10 acts in concert with the tissue gripping device 26 and thereby, the flange 16 at the distal end of the sealing device 10 is disengaged from the patient's skin.

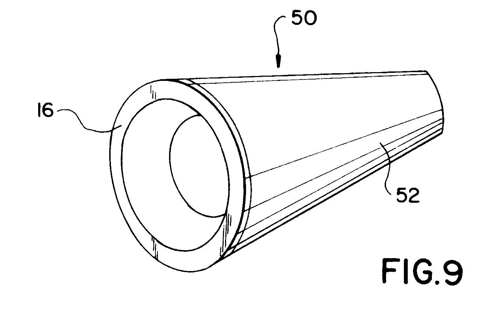

Another embodiment of the sealing device 50 is shown in Fig. 9. The sealing device includes a flange 16 at its distal end as in the previous embodiment shown in Figs. 1-8. However, in the embodiment shown in Fig. 9, the sealing device 50 includes a generally frustoconical member 52. The generally frustoconical member 32 has a smaller diameter at its proximal end than at its distal end. A flange 16 is located at the distal end of the generally frustoconical member 52 that is positionable against a patient's body in the same manner as the embodiment shown in Figs. 1-8. It is contemplated that the generally frustoconical member 52 may be used with or as part of a cannula 18 or trocar assembly 24, 42, 46 similar to the embodiment shown in Figs. 1-8. Thus, the generally frustoconical member 52 may include an inner diameter at its proximal end defined by a neck dimensioned to fit securely about a cannula. Further, the generally frustoconical member 52 may include an inner circumference at its proximal end defined by a ridge which cooperates with a receiving indentation or groove in the proximal end of a cannula. Moreover, the generally frustoconical member 52 may be used with or as part of a trocar assembly.

Embodiments of the invention are defined within the scope of the claims below.

## Claims

1. A sealing means for use with a cannula to inhibit leakage of gas and fluids about an incision, said sealing means comprising:
a substantially cylindrical member positioned about said cannula including a flange at a distal end thereof, said flange being positionable against a patient's body.

2. A sealing means according to claim 1, wherein said substantially cylindrical member is longitudinally deformable.

3. A sealing means according to claim 1 or 2, wherein said substantially cylindrical member includes a plurality of bulbous portions.

4. A sealing means according to claim 3, wherein the number of said bulbous portions in said plurality can be varied.

5. A sealing means according to any one of the preceding claims, wherein said sealing means is couplable to said cannula by means of an adhesive.

6. A sealing means according to any one of the preceding claims wherein said substantially cylindrical member is angularly adjustable so that the plane of the distal flange can pivot relative to the long axis of the cylindrical member.

7. A sealing means according to claim 6, wherein said substantially cylindrical member has an inside diameter big enough to allow angular movements of said cannula with respect to the plane of said flange.

8. A sealing means according to claim 7, wherein said substantially cylindrical member is of a generally frustoconical shape with a smaller diameter at its proximal end than at said distal end.

9. A sealing means according to any one of the preceding claims, wherein said substantially cylindrical member includes at its proximal end a mating formation along an inner circumference that couples with a complementary mating formation on said cannula.

10. A sealing means according to any one of the preceding claims, wherein said substantially cylindrical member is resiliently longitudinally compressible.

11. A sealing means according to any one of the preceding claims wherein the flange is adapted to form a substantially continuous seal against the skin of the patient's body.

12. A cannula including a sealing means as claimed in any one of the preceding claims.

13. A cannula comprising:
a tubular body portion;
a sealing means concentrically positioned about said tubular body portion; said sealing means including a substantially cylindrical member having a flange at a distal end thereof, said flange being positionable against a patient's body.
